# EUROPEAN PATENT APPLICATION

(11) **EP 2 546 350 A1**
(43) Date of publication of application: **16.01.2013**
(21) Application number: 11753499.0
(22) Date of filing: 11.03.2011
(51) Int. Cl.: C12P 7/40, C07C 59/76, A01H 5/00, C12N 15/29

(54) **PROCESS FOR PRODUCING KODA USING LEMNA PAUCICOSTATA**

(30) Priority: 11.03.2010 JP 2010055166
(71) Applicant: Shiseido Company, Ltd., Chuo-ku Tokyo 104-8010 (JP)
(72) Inventor: YOKOYAMA, Mineyuki, Yokohama-shi Kanagawa 236-8643 (JP); BEPPU, Toshio, Uenohara-shi Yamanashi 409-0193 (JP)
(74) Representative: Henkel, Breuer & Partner
(86) International application number: PCT/JP2011/055842
(87) International publication number: WO 2011/111841

(57) **Abstract**

The genes of a novel lipoxygenase and a novel allene oxide synthase derived from Lemna paucicostata strain SH are identified, and a plant growth regulator (KODA) is produced with high yield by using a Lemna paucicostata strain that expresses said lipoxygenase and said allene oxide synthase.

## Description

### [Technical Field]

The present invention relates to a method of preparing a plant hormone having a structure represented by the following Formula (I) (common name: 9-hydroxy-10-oxo-cis-12(Z),15(Z)-octadecadienoic acid, herein after referred to as KODA):

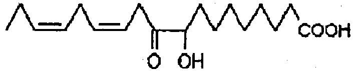

### [Background Art]

KODA is known to be a plant hormone having a plant floral bud-formation promoting activity, a plant activating activity, and a plant growth regulating activity incorporating these activities (Japanese Unexamined Patent Publication (Kokai) No. 9-295908, Japanese Unexamined Patent Publication (Kokai) No. 11-29410, Japanese Unexamined Patent Publication (Kokai) No. 2001-131006, Japanese Unexamined Patent Publication (Kokai) No. 2009-17829). KODA is known to be present in various plant species, and Lemna paucicostata that was subjected to stress is known to release KODA at a very high level (a few hundred-fold higher) compared to other plants. Utilizing this property, KODA can be prepared using an extraction method in which KODA is obtained by extracting from Lemna paucicostata, a species of the family Lemnaceae. As other production methods, KODA can be prepared by using an enzymatic method in which α-linolenic acid (common name: cis-9,12,15-octadecatrienoic acid), which is an unsaturated fatty acid, is subjected to enzymes such as position 9-product specific lipoxygenase (LOX), allene oxide synthase (AOS) in accordance with the fatty acid metabolic pathway in a plant, or by using a chemical synthetic method in which generally known chemical synthetic methods are used. These production methods are disclosed in Japanese Unexamined Patent Publication (Kokai) No. 11-29410.

Since KODA is a plant hormone having a plant growth regulating activity, its application in the agricultural filed is promising. When used in agriculture, unlike pharmaceuticals, it cannot be put into practical use unless produced at low cost and in large quantities.

In an enzymatic method that employs α-linolenic acid as a starting substance, as described below, KODA can be prepared by acting 9-specific lipoxygenase (LOX) on α-linolenic acid as the substrate, thereby introducing a hydroperoxy group (-OOH) at position 9, and then acting allene oxide synthase (AOS) thereon. 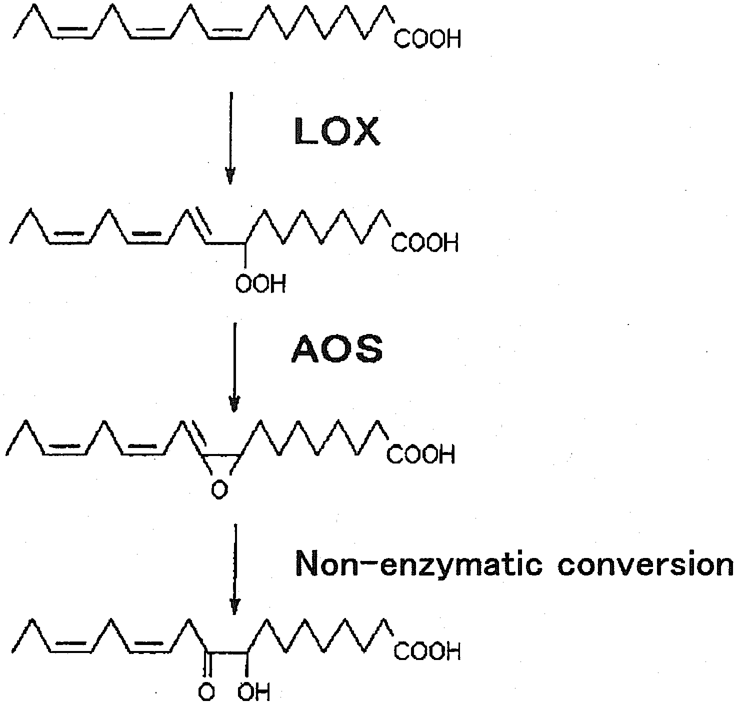 However, position 9-specific lipoxygenase is not commercially available, and even the extraction from a plant requires a lot of time and effort on obtaining and processing materials. Also, the activity of position 9-specific lipoxygenases obtained to date was low. Furthermore, when cDNA of position 9-specific lipoxygenase known to date was expressed in Escherichia coli, most of them are turned out to be insoluble, and thus it was difficult to obtain active protein at large quantities.

Allene oxide synthase is an enzyme having an activity of converting hydroperoxylated fatty acid to allene oxide, and since allene oxide is unstable, it is non-enzymatically converted to α-ketol form. AOS is an enzyme present in plants, animals, and yeast, and in plants, it is present in throughout the angiosperms. However, allene oxide synthase generally has a suicide substrate-like property, and thus when the substrate concentration is increased, the amount of product conversely decreases. Due to the drawbacks of the lipoxygenase and allene oxide synthase, the enzymatic method was not suitable for a large scale production. On the other hand, in chemical synthesis, it was difficult to attain the low cost that was desired at the agricultural filed.

On the other hand, while the conventional extraction method was carried out by culturing Lemna paucicostata 441 strain that are known to produce a floral bud-inducing substance at high efficiency, even the use of such strains could not produce a sufficient amount of KODA. Thus, there has been a need for a method of preparing KODA at low cost and in large quantities.

### [Summary of Invention]

### [Technical Problem]

An object of the present invention is to provide a method of preparing KODA in which the yield of KODA has been improved.

### [Solution to Problem]

In intensive research to solve the above problem, the present inventors conducted the screening of various strains of Lemna paucicostata. As a result, the present inventors have found that compared to other Lemna paucicostata strains, Lemna paucicostata SH strain produces an extremely high level of KODA.

### [Advantageous Effects of Invention]

Based on the above finding, the present inventors provide a method of preparing KODA at a high yield by using Lemna paucicostata SH strain as the starting substance in a method of preparing KODA based on the extraction method.

Furthermore, the present inventors have focused on the metabolic pathway of Lemna paucicostata SH strain which is a high KODA-producing strain, and have succeeded for the first time in identifying the gene sequence (SEQ ID NO: 1) of position 9-specific lipoxygenase and the gene sequence (SEQ ID NO: 2) of allene oxide synthase in the Lemna paucicostata. The base sequences of said SEQ ID NO: 1 and SEQ ID NO: 2 are sequences both derived from Lemna paucicostata SH strain.

Thus, the present inventors provide a method of preparing KODA based on the extraction method wherein KODA can be produced at a high yield by using a Lemna paucicostata strain having a gene consisting of a DNA consisting of a sequence represented by SEQ ID NO: 1 and/or SEQ ID NO: 2, or a DNA substantially identical to the above DNA,

The present invention also provides KODA produced by the production method using a high KODA-producing Lemna paucicostata strain described above.

### [Brief Description of Drawings]

[Fig. 1]

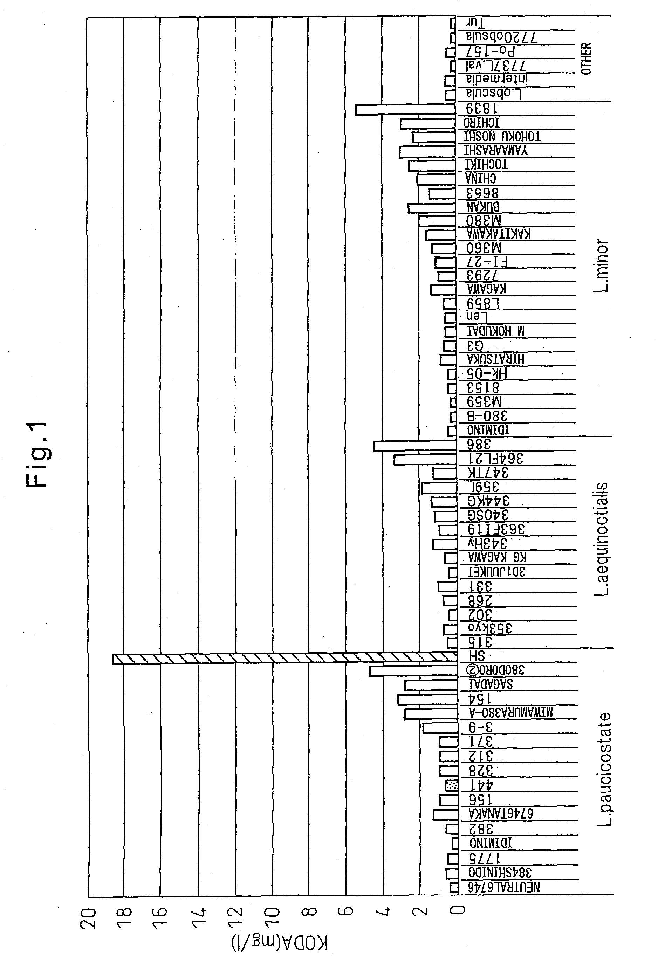

   Fig. 1 is a graph showing the amount of KODA produced for 62 strains of Lemna paucicostata.
[Fig. 2A]

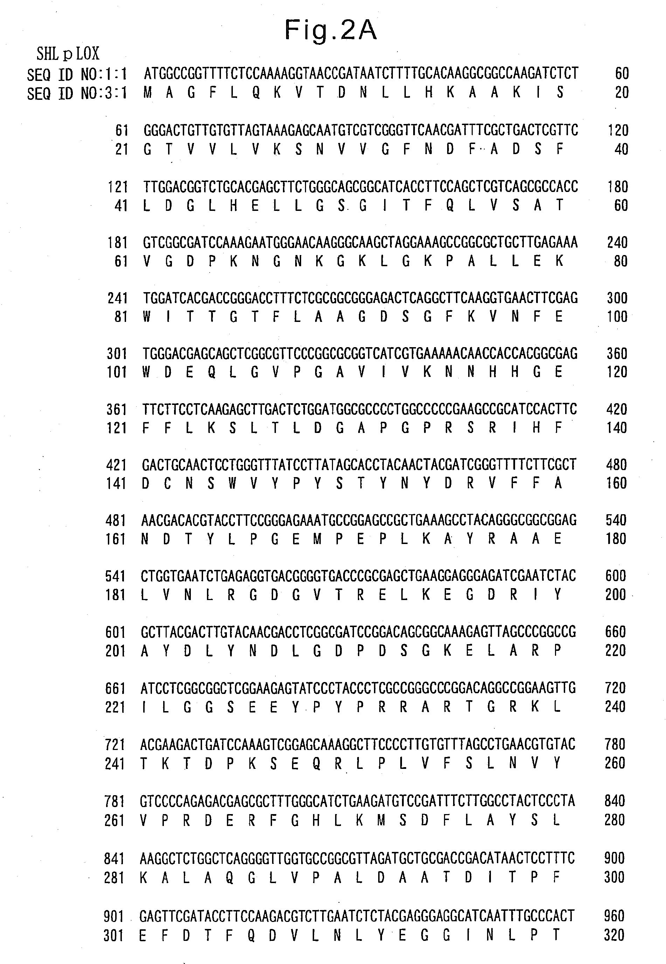

   Fig. 2A shows the gene sequence of the LOX gene of Lemna paucicostata SH strain.
[Fig. 2B]

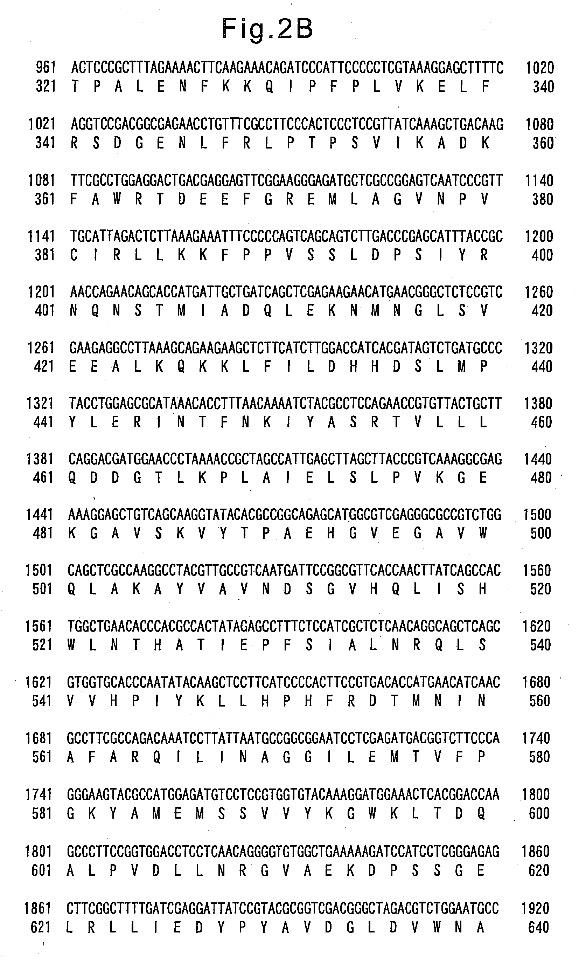

   Fig. 2B shows the continuation of gene sequence of Fig. 2A.
[Fig. 2c]

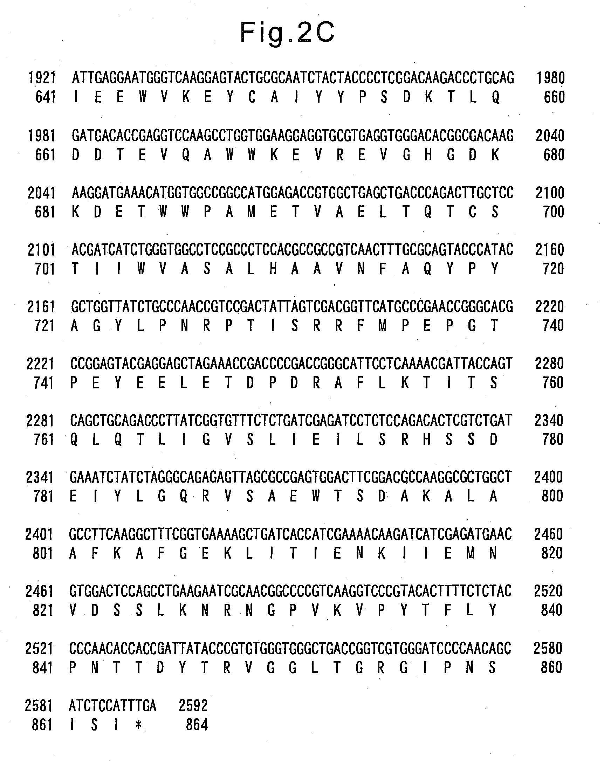

   Fig. 2C shows the continuation of gene sequence of Fig. 2B.
[Fig. 3A]

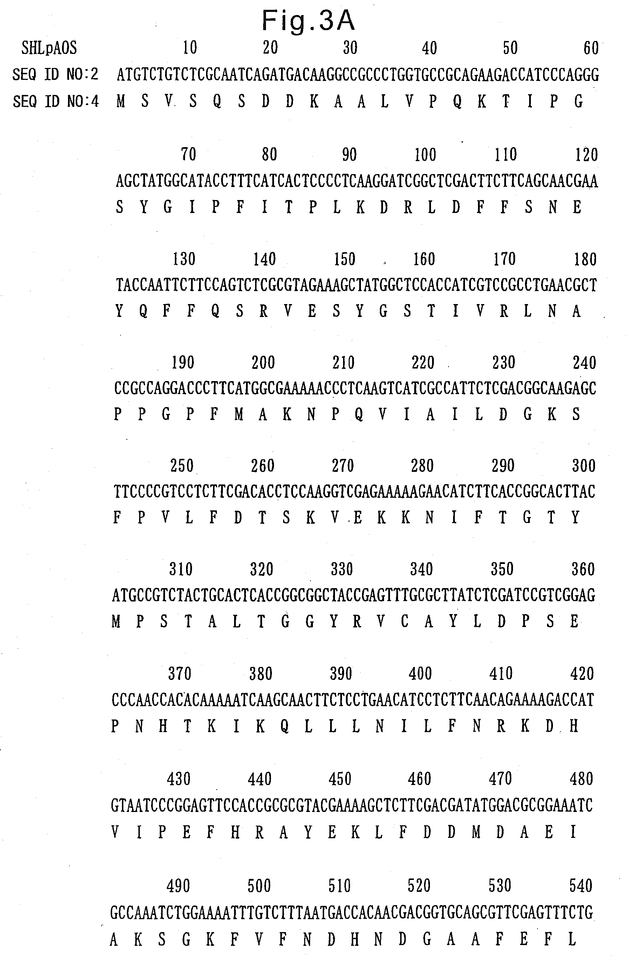

   Fig. 3A shows the gene sequence of the AOS gene of Lemna paucicostata SH strain.
[Fig. 3B]

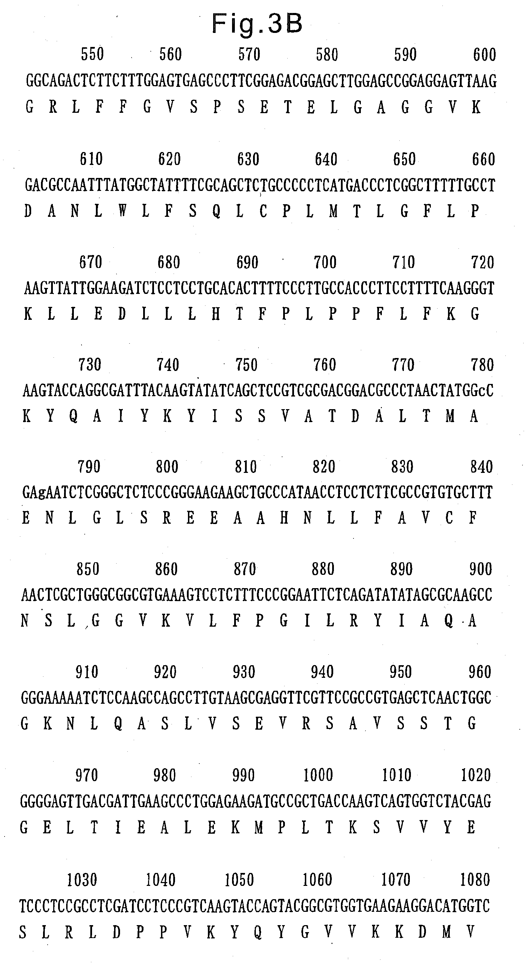

   Fig. 3B shows the continuation of gene sequence of Fig. 3A.
[Fig. 3c]

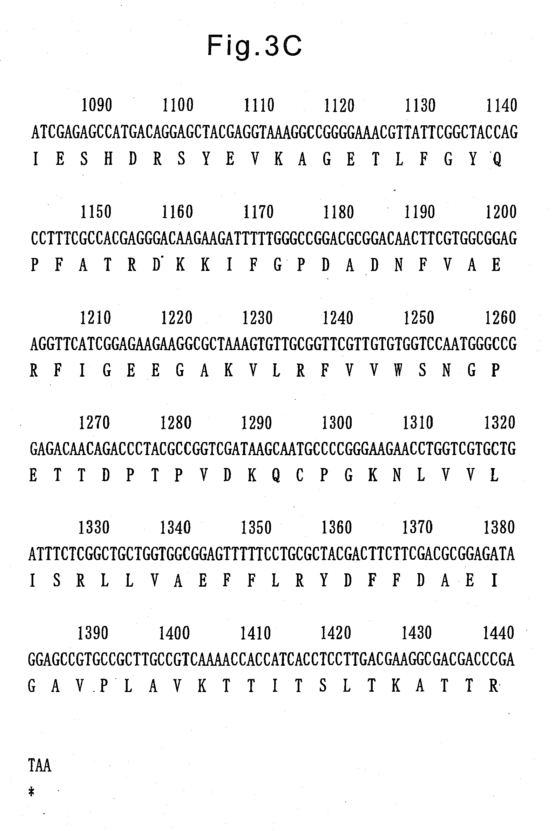

   Fig. 3C shows the continuation of gene sequence of Fig. 3B.
[Fig. 4]

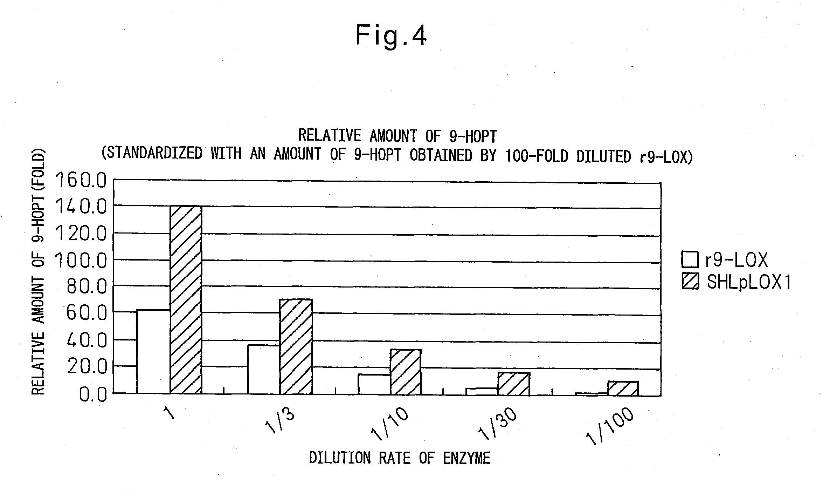

   Fig 4 is a graph showing the comparison of the activity of a rice plant LOX (r9-LOX) and LOX of Lemna paucicostata SH strain expressed in Escherichia coli.
[Fig. 5]

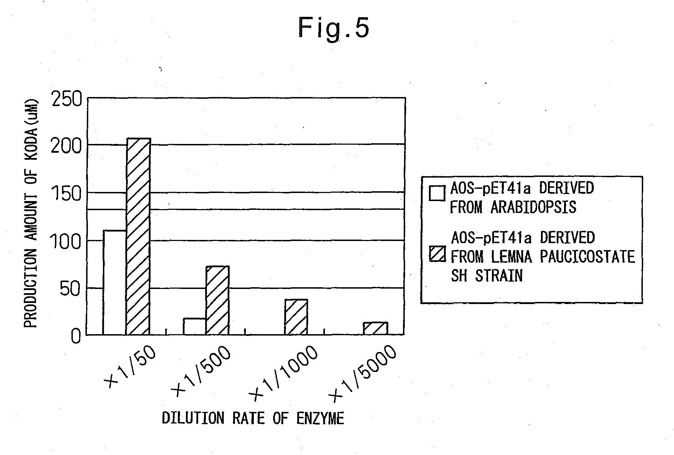

   Fig. 5 is a a graph showing the comparison of the activity of AOS of Arabidopsis thaliana and AOS of Lemna paucicostata SH strain expressed in Escherichia coli.

### [Description of Embodiments]

The method of preparing KODA of the present invention comprises applying stress to a specific high KODA-producing Lemna paucicostata strain, extracting KODA with a solvent from said stressed Lemna paucicostata strain, and purifying KODA.

In an embodiment of the present invention, the specific high KODA-producing Lemna paucicostata strain to be used in the present invention is a strain that expresses a protein encoded by a DNA identical with or substantially identical with a DNA consisting of a base sequence represented by SEQ ID NO: 1 and/or a DNA that is identical with or substantially identical with a DNA comprising a base sequence represented by SEQ ID NO: 2. Furthermore, the specific high KODA-producing Lemna paucicostata strain of the present invention to be used in the present invention is a strain that expresses a protein identical with or substantially identical with a protein consisting of an amino acid sequence represented by SEQ ID NO: 3, and/or a protein identical with or substantially identical with a protein consisting of an amino acid sequence represented by SEQ ID NO: 4. SEQ ID NO: 3 is the amino acid sequence of position 9-product specific lipoxygenase derived from Lemna paucicostata SH strain, and SEQ ID NO: 4 is the amino acid sequence of allene oxide synthase derived from Lemna paucicostata SH strain.

Since KODA is prepared from linolenic acid by the acting of LOX and AOS in the plant, a strain that expresses LOX and/or AOS identical to or substantially identical to LOX and/or AOS of the high KODA-producing Lemna paucicostata SH strain is considered to have a high KODA productivity similarly to the Lemna paucicostata SH strain. As used herein the term "substantially identical DNA" refers to a DNA that has a 70% identity with the reference DNA, and that encodes a protein, when transcribed and translated, having the same enzyme activity as that of a protein produced by the transcription and translation of the reference DNA (the LOX activity in the case of a DNA consisting of a base sequence represented by SEQ ID NO: 1, and the AOS activity in the case of a DNA consisting of a base sequence represented by SEQ ID NO: 2). The identity may preferably be at least 80%, at least 90%, at least 95%, at least 97%, at least 98%, at least 99%, at least 99.5%, or at least 99.9%.

Also, "substantially identical DNA" refers to a DNA that can hybridize to a DNA consisting of a base sequence complementary to the reference DNA under a high stringent condition, and that encodes a protein having the same enzyme activity as that of a protein encoded by the reference DNA (the LOX activity in the case of a DNA comprising a base sequence represented by SEQ ID NO: 1, and the AOS activity in the case of a DNA comprising a base sequence represented by SEQ ID NO: 2). Hybridization can be carried out by a known method or a method based on said method, such as a method described in J. Sambrook et al., Molecular Cloning, 2nd., Cold Spring Harbor Laboratory Press, 1989, and a high stringent hybridization condition refers to, for example, a NaCl concentration of about 10-40 mM, preferably about 20 mM, and a temperature of about 50-70°C, preferably about 60-65°C. Furthermore, the present invention also relates to a DNA fragment of said DNA sequence, said fragment encoding a protein having the enzyme activity of the protein encoded by the original DNA.

As used herein "a substantially identical protein" refers to a protein that comprises an amino acid sequence in which one or a few amino acids have been deleted, substituted or added in the amino acid sequence of the reference protein, and that has the activity of the reference protein. Furthermore, "a substantially identical protein" refers to a protein that comprises a sequence having an identity of 98% with the reference amino acid sequence, and that has the activity of the reference protein. The identity may preferably be at least 99%, at least 99.5%, or at least 99.9%.

The method of preparing KODA of the present invention utilizes an extraction method. Specifically, a homogenate of Lemna paucicostata is centrifuged (8000×g, for about 10 minutes), and among the supernatant and the precipitate obtained, the supernatant is removed, and the rest is used in the next step as a KODA-containing fraction. Using such a fraction as the starting substance, KODA can be isolated and purified. In order to promote the production of KODA from Lemna paucicostata, it may be preferred to apply a specific stress described below to Lemna paucicostata before centrifugation.

As a preferred starting substance in terms of preparation efficiency, there can be mentioned an aqueous solution obtained after Lemna paucicostata was allowed to float or immersed. This aqueous solution may not be specifically limited as long as Lemna paucicostata can grow therein. Specific examples of the aqueous solution will be described in the Examples described below.

The immersing time may be about 2-3 hours at room temperature, but should not be limited. When KODA is prepared by this method, it may be preferred in terms of production efficiency to apply to Lemna paucicostata a specific stress that can induce KODA.

Specifically, as a specific stress, there can be mentioned a drying stress, a heat stress, an osmotic stress and the like. The drying stress can be applied by, for example, allowing Lemna paucicostata to be left spreaded on a dry filter paper at a low humidity (preferably a relative humidity of 50% or less) and at room temperature, preferably about 24-25°C. The drying time in this case is roughly 20 seconds or more, preferably 5 minutes or more, and more preferably 15 minutes or more.

The heat stress may be applied by, for example, immersing Lemna paucicostata in a warm water. The temperature of the warm water may be 40-65°C, preferably 45-60°C, and more preferably 50-55°C. As the time of immersing in the warm water, roughly 5 minutes may be sufficient, and when the temperature is relatively low, for example when Lemna paucicostata is treated in a warm water of about 40°C, it is preferable to treated for 2 hours or more. After the above heat stress treatment, Lemna paucicostata may preferably be quickly returned to cold water.

The osmotic stress may be applied by, for example, contacting Lemna paucicostata with a high osmotic solution such as a high-concentration sugar solution. The sugar concentration at this time, in the case of a mannitol solution, may preferably be 0.3 M or more, and preferably 0.5 M or more. When, for example, a 0.5 M mannitol solution is used, the treating time may preferably be one minute or more, and preferably 3 minutes or more. Thus, the desired starting substance containing KODA of the present invention can be prepared.

Then, the starting substance prepared as described above may be subjected to the separation and purification treatment as described below to prepare the desired KODA. The separation means shown in this specification is only for illustrative purposes, and a separation means for producing KODA from the above starting substance should not be limited to the separation means.

First, it may be preferred that the above starting substance is subjected to solvent extraction to extract a component containing KODA of the present invention. The solvent to be used in such solvent extraction should not be specifically limited, and for example chloroform, ethyl acetate, butanol etc., may be used. Among these solvents, chloroform is preferred since it can remove impurities relatively easily.

The oil phase obtained by this solvent extraction may be washed and concentrated by a commonly known method, and subjected to high performance liquid chromatography (HPLC) using a reverse phase partition chromatography with an ODS (octadecylsilane) column for identification and isolation of a fraction having a floral bud-inducing activity to isolate KODA of the present invention. Depending on the properties of the starting substance, other generally known separation means such as ultrafiltration, gel filtration chromatography etc., may be used in combination. Examples

### Example 1: Screening of a high KODA-producing Lemna paucicostata strain

62 types of Lemna paucicostata harvested from different places were prepared and were subcultured in the 1/2-diluted Hutner's medium under continuous illumination of daylight fluorescent light at 24-25 °C. The 1/2-diluted Hutner's medium comprises the following ingredients:

| | |
|---|---|
| Sucrose | 10 g/l |
| K₂HPO₄ | 200 mg/l |
| NH₄NO₃ | 100 mg/l |
| EDTA free acid | 250 mg/l |
| Ca(N03)·4H₂O | 176 mg/l |
| MgSO₄·7H₂O | 250 mg/l |
| FeSO₄·7H₂O | 12.4 mg/l |
| MnCl₂·4H₂O | 8.92 mg/l |
| ZnSO₄·7H₂O | 32.8 mg/l |
| Na₂MoO₄·2H₂O | 12.6 mg/l |
| H₃BO₃ | 7.1 mg/l |
| Co(NO₃)·6H₂O | 0.1 mg/l |
| CuSO₄·5H₂O | 1.97 mg/l |
| and pH was adjusted to 6.2-6.5 with KOH (50%). | |

The grown Lemna paucicostata was spreaded on a filter paper, and after incubating for 2 hours, it was immersed in water for 1 hour. The water was subjected to high performance liquid chromatography (HPLC; column: TYPE UG120 5 µm, SIZE: 4.6 mm I.D.x250 mm; guard filter: INERTSTL 4.6mmx50 mm; eluent: 50% acetonitrile+0.1% trifluoroacetic acid; condition: absorption wavelength 210 λ(nm), flow rate: 1 ml/min, column temperature: 40°C) to analyze the concentration of KODA. The mean of the production amount of KODA in all Lemna paucicostata strains was 4.97 µM. Among them, Lemna paucicostata SH strain produced 60.2 µM of KODA, which is about 12-times higher than the mean amount of KODA produced, giving a very high production amount (Fig. 1).

### Example 2: Cloning of lipoxygenase derived from Lemna paucicostata SH strain and measurement of the activity thereof

From Lemna paucicostata (SH strain), total RNA was extracted by using the RNeasy Plant Mini Kit (QIAGEN), and then cDNA was synthesized by using 1.8 µg of total RNA as the template in LongRange 2 Step RT-PCR Kit (QIAGEN).
Then, degenerate PCR (PCR condition: initial denaturation at 94°C for 3 minutes; a cycle comprising 94°C for 0.5 minute, 47°C for 0.5 minute, and 72°C for 1.3 minute is carried out for 39 times) was carried out by using the cDNA as the template, and using the following degenerate primers (LpDPf, LpDPr) to obtain a partial sequence of 9-lipoxygenase of interest.
LpDPf: 5'-GCITGGMGIAGIGAYGARGARTTY-3' (SEQ ID NO: 5)
LpDPr: 5'-GCRTAIGGRTAYTGICCRAARTT-3' (SEQ ID NO: 6)
wherein, I represents inosine.
After the base sequence of said partial sequence was determined, BLAST search was carried out based on the sequence information obtained. As a result, the sequence obtained exhibited a high homology with LOX derived from a plurality of known plants (75% with Corylus avellana, 74% with Actinidia deliciosa, 75% with Solanum tuberosum, 76% with Oryza sativa, 74% with Nicotiana tabacum, 75% with Cucumis sativus, 73% with Arabidopsis thaliana, etc.).

Based on this sequence information, primers for the following 3' or 5' RACE method (Rapid Amplification of cDNA end) were constructed, and the full-length sequence was determined by the 3' or 5' RACE method (Fig. 2).
SH-3'-TP: 5'-AGCTCTTCATCTTGGACC-3' (SEQ ID NO: 7)
SH-5'-TP: 5'-TTTCATCCTTCTTGTCGC-3' (SEQ ID NO: 8)

The sequence of SHLpLOSX obtained was introduced into a vector (pET23d, Novagen) for protein expression, and then transformed into Escherichia coli (BL21(DE3), Novagen), so as to allow the expression of SHLpLOX protein. Using this SHLpLOX protein, the activity test in KODA production was carried out.

In the activity test in KODA production, to an aqueous solution comprising 5 mM linolenic acid solution (dissolved in 0.1% Tween 80 solution; 25 µl), 0.2 M sodium phosphate buffer (pH 7, 10 µl), and distilled water (5 µl), an enzyme solution (10 µl) was added, and allowed to react at room temperature for 30 minutes. After the reaction was over, the reaction mixture was subjected to HPLC to determine the site specificity and the amount of linolenic acid hydroperoxide to be formed. HPLC analysis was carried out with a column: Capcell pak C-18 UG120 (4.6x250 mm, Shiseido), by using column temperature: 40°C, mobile phase: 50% acetonitrile solution (0.02% TFA), flow rate: 1 ml/min, detection wavelength: 210 nm. In this case, as a position 9-specific control enzyme, r9-LOX1 which is a position 9-specific lipoxygenase derived from rice germ. It was revealed that novel LOX obtained from Lemna paucicostata SH strain is a position 9-product specific lipoxygenase having a much higher activity than r9-LOX1 that had been considered to be the most potent among the known 9-product specific lipoxygenases (Fig. 4).

Among the novel LOXs derived from Lemna paucicostata, SHLpLOX that had the highest production amount of linolenic acid-9-hydroperoxide was subjected to kinetic analysis. Using a reaction mixture comprising 40 mM phosphate buffer (pH 6.0) and 0.1% Tween 80, the analysis was carried out at a temperature of 25°C. The substrate α-linolenic acid was tested at the substrate concentration of 10-100 µM. 100 µl of the reaction mixture was added into a cuvette, and absorbance at 234 nm was scanned over time for 10 minutes at an interval of 15 seconds using the SmartSpec Plus Spectrophotometer (Bio-Rad). The amount of the reaction product was calculated from the A₂₃₄ determined (e=25,000). Kinetic parameters were determined using the Hanes-Woolf plot ([S]/v versus [S] plot). As shown in Table 1, the result shows that the Kₘ value which is an affinity parameter for substrate is lower in SHLpLOX than in r9-LOX1, thereby indicating that SHLpLOX had a high affinity for the substrate α-linolenic acid. The maximum reaction velocity Vₘₐₓ is almost comparable between r9-LOX and SHLpLOX, while the k_{cat} value which is the number of reactions per unit time was higher in SHLpLOX than in r9-LOX1. The k_{cat}/Kₘ value which is an index of enzyme activity was about 1.6-fold higher in SHLpLOX than in r9-LOX1. This revealed that the novel 9-LOX derived from Lemna paucicostata SH strain is a very highly active 9-LOX.

**[Table 1]**

| Kinetic parameters of SHLpLOX and r9-LOX1 | | | | |
|---|---|---|---|---|
| | Kₘ (µM) | Vmax (µmol min⁻¹) | k_{cat} (min⁻¹) | K_{cat}/Kₘ (×10⁵M⁻¹min⁻¹) |
| LpLOX-SH | 19.2±4.1 | 3.0±0.1 | 768.2±32.3 | 41.4±10.0 |
| r9-LOX1 | 22.9±1.6 | 2.8±0.6 | 614.1±124.6 | 26.7±4.2 |

### Example 3: Cloning of the AOS gene derived from Lemna paucicostata SH strain and the activity measurement thereof

Total RNA was extracted from Lemna paucicostata (SH strain), and cDNA was synthesized by the RT-PCR method. Then, using the synthesized cDNA as the template and using the primers derived from Arabidopsis thaliana as described below, a partial sequence information of allene oxide synthase derived from the SH strain (SHLpAOS) was obtained by setting the annealing temperature for PCR at a low temperature of 45°C.
AOS-Forward: 5'-GGAACTAACCGGAGGCTACCG-3' (SEQ ID NO: 9)
AOS-Reverse: 5'-CCGTCTCCGGTCCATTCGACCACAA-3' (SEQ ID NO: 10)

Based on this sequence information, the full-length sequence was determined by the 3' or 5' RACE (Rapid Amplification of cDNA end) method. As a result, a novel AOS homolog (nucleotide sequence: 1443 bp, amino acid sequence: 480 aa, deduced molecular weight: 53.3 KDa) of one sequence was obtained from the SH strain (Fig. 3).

The sequence of SHLpAOS obtained was introduced into a vector (pET41a, Novagen) for protein expression, and then transformed into Escherichia coli (BL21(DE3), Novagen), thereby allowing the SHLpAOS protein to be expressed. Using this SHLpAOS protein, the activity test in KODA production was carried out.

In the activity testing in KODA production, 5 mM linolenic acid solution (dissolved in 0.1% Tween 80 solution) was prepared, and reacted with lipoxygenase extracted from rice germ at pH 7 at room temperature for 10 minutes to synthesize 9-hydroperoxylinolenic acid (9-HPOT) reaction mixture. To 20 µl of this 9-HPOT reaction mixture, 0.32 ng of the SHLpAOS protein or A. thaliana-derived AOS (AtAOS) protein was added and reacted at room temperature for 10 minutes. After the reaction was over, the reaction was terminated by a heat treatment at 50°C for 3 minutes. 10 µl of this solution was analyzed by HPLC. HPLC analysis was carried out with a column: Capsule pack C-18 UG120 (4.6x250 mm), and using mobile phase: 50% acetonitrile solution (0.02% TFA), flow rate: 1 ml/min, detection wavelength: 210 nm.
As a result, the SHLpAOS protein had an activity nearly 7-fold higher than the AtAOS protein (Fig. 5).

SHLpAOS obtained from Lemna paucicostata SH strain was subjected to kinetic analysis. Using a reaction mixture comprising 40 mM phosphate buffer (pH 7.5) and 1% EtOH, the reaction was carried out at a temperature of 25°C. The substrate 9-HPOT was tested at the substrate concentration of 5-53 µM. The substrate 9-HPOT was added as an EtOH solution, and the final concentration of EtOH was adjusted to 1%. 100 µl of the reaction mixture was added into a cuvette, and a decrease in absorbance at 234 nm was scanned over time for 1 minute at an interval of 2 seconds using the SmartSpec Plus Spectrophotometer (Bio-Rad). From the A₂₃₄ determined, the amount of the reaction product was calculated (e=25,000). Kinetic parameters were determined using the Hanes-Woolf plot ([S]/v versus [S] plot). As shown in Table 2, the result indicates that the Kₘ value is significantly lower in SHLpAOS than in AtAOS, and had an about 5-fold higher affinity for 9-HPOT in SHLpAOS than in AtAOS. The Vₘₐₓ was about 2.8-fold higher in SHLpAOS than in AtAOS. The K_{cat} value was also about 2.8-fold higher in SHLpAOS than in AtAOS, indicating that reaction turnover is occurring very efficiently. The k_{cat}/Kₘ value was about 14-fold higher in SHLpAOS than in AtAOS. It is believed that compared to AtAOS, SHLpAOS is a very useful AOS in the practical production of KODA. The above revealed that SHLpAOS cloned from Lemna paucicostata SH strain is an AOS having a very high activity that has not been reported before.

**[Table 2]**

| Kinetic parameters of SHLpLOX and AtAOS | | | | |
|---|---|---|---|---|
| | Kₘ (µM) | Vₘₐₓ (µmol sec⁻¹) | k_{cat} (sec⁻¹) | k_{cat}/Kₘ (×10⁶M⁻¹sec⁻¹) |
| LpLOX-SH | 7.1±1.9 | 4.7±0.3 | 709.2±44.3 | 99.0±23.7 |
| AtAOS | 36.3±1.3 | 1.7±0.03 | 255.5±4.8 | 7.0±3.8 |

## Claims

1. A method of preparing a compound represented by the following formula: 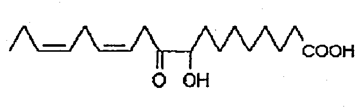 comprising applying stress to Lemna paucicostata strain that expresses a protein encoded by a DNA selected from the group consisting of the following (a) to (e) or a protein encoded by a DNA selected from the group consisting of the following (i) to (v):
(a) a DNA consisting of a base sequence of SEQ ID NO: 1;
(b) a DNA that hybridizes to a DNA consisting of a base sequence complementary to a DNA consisting of a base sequence of SEQ ID NO: 1 under a high stringent condition, and that encodes a position 9-product specific lipoxygenase active form;
(c) a DNA that encodes a protein comprising an amino acid sequence of SEQ ID NO: 3;
(d) a DNA encoding a protein that comprises a sequence having a homology of at least 99% with an amino acid sequence of SEQ ID NO: 3, and that has a position 9-product specific lipoxygenase activity; and
(e) a DNA encoding a protein that comprises an amino acid sequence in which one or a few amino acids have been deleted, substituted or added in an amino acid sequence of SEQ ID NO: 3, and that has a position 9-product specific lipoxygenase activity; and
(i) a DNA comprising a base sequence of SEQ ID NO: 2;
(ii) a DNA that has a homology of at least 90% with a DNA comprising a base sequence of SEQ ID NO: 2 and that encodes an allene oxide synthase active form;
(iii) a DNA that hybridizes under a high stringent condition to a DNA comprising a base sequence complementary to a DNA comprising a base sequence of SEQ ID NO: 2 and that encodes an allene oxide synthase active form;
(iv) a DNA encoding a protein that comprises an amino acid sequence of SEQ ID NO: 4; and
(v) a DNA encoding a protein that comprises an amino acid sequence in which one or a few amino acids have been deleted, substituted or added in an amino acid sequence of SEQ ID NO: 4, and that has an allene oxide synthase activity,
extracting the above compound with a solvent from said stressed Lemna paucicostata strain, and
purifying the compound.

2. The method according to claim 1, wherein the Lemna paucicostata strain expresses lipoxygenase encoded by a DNA comprising a base sequence of SEQ ID NO: 1 and allene oxide synthase encoded by a DNA comprising a base sequence of SEQ ID NO: 2.

3. The method according to claim 1 or 2 wherein the stress is selected from the group consisting of a drying stress, a heat stress and an osmotic stress.

4. The method according to any one of claims 1 to 3
wherein the solvent is selected from the group consisting of chloroform, ethyl acetate, ether and butanol.


SEQUENCE LISTING

<110>  Shiseido Company, Ltd.

<120>  Method for preparing KODA using Lemna paucicostata

<130>  Z569-PCT

<150>  JP 2010-055166

<151>  2010-03-11

<160>  10

<170>  PatentIn version 3.1

<210>  1
<211>  2592
<212>  DNA
<213>  Lemna paucicostata

<400>  1
atggccggtt ttctccaaaa ggtaaccgat aatcttttgc acaaggcggc caagatctct      60

gggactgttg tgttagtaaa gagcaatgtc gtcgggttca acgatttcgc tgactcgttc     120

ttggacggtc tgcacgagct tctgggcagc ggcatcacct tccagctcgt cagcgccacc     180

gtcggcgatc caaagaatgg gaacaagggc aagctaggaa agccggcgct gcttgagaaa     240

tggatcacga ccgggacctt tctcgcggcg ggagactcag gcttcaaggt gaacttcgag     300

tgggacgagc agctcggcgt tcccggcgcg gtcatcgtga aaaacaacca ccacggcgag     360

ttcttcctca agagcttgac tctggatggc gcccctggcc cccgaagccg catccacttc     420

gactgcaact cctgggttta tccttatagc acctacaact acgatcgggt tttcttcgct     480

aacgacacgt accttccggg agaaatgccg gagccgctga aagcctacag ggcggcggag     540

ctggtgaatc tgagaggtga cggggtgacc cgcgagctga aggagggaga tcgaatctac     600

gcttacgact tgtacaacga cctcggcgat ccggacagcg gcaaagagtt agcccggccg     660

atcctcggcg gctcggaaga gtatccctac cctcgccggg cccggacagg ccggaagttg     720

acgaagactg atccaaagtc ggagcaaagg cttccccttg tgtttagcct gaacgtgtac     780

gtccccagag acgagcgctt tgggcatctg aagatgtccg atttcttggc ctactcccta     840

aaggctctgg ctcaggggtt ggtgccggcg ttagatgctg cgaccgacat aactcctttc     900

gagttcgata ccttccaaga cgtcttgaat ctctacgagg gaggcatcaa tttgcccact     960

actcccgctt tagaaaactt caagaaacag atcccattcc ccctcgtaaa ggagcttttc    1020

aggtccgacg gcgagaacct gtttcgcctt cccactccct ccgttatcaa agctgacaag    1080

ttcgcctgga ggactgacga ggagttcgga agggagatgc tcgccggagt caatcccgtt    1140

tgcattagac tcttaaagaa atttccccca gtcagcagtc ttgacccgag catttaccgc    1200

aaccagaaca gcaccatgat tgctgatcag ctcgagaaga acatgaacgg gctctccgtc    1260

gaagaggcct taaagcagaa gaagctcttc atcttggacc atcacgatag tctgatgccc    1320

tacctggagc gcataaacac ctttaacaaa atctacgcct ccagaaccgt gttactgctt 1380

caggacgatg gaaccctaaa accgctagcc attgagctta gcttacccgt caaaggcgag 1440

aaaggagctg tcagcaaggt atacacgccg gcagagcatg gcgtcgaggg cgccgtctgg 1500

cagctcgcca aggcctacgt tgccgtcaat gattccggcg ttcaccaact tatcagccac 1560

tggctgaaca cccacgccac tatagagcct ttctccatcg ctctcaacag gcagctcagc 1620

gtggtgcacc caatatacaa gctccttcat ccccacttcc gtgacaccat gaacatcaac 1680

gccttcgcca gacaaatcct tattaatgcc ggcggaatcc tcgagatgac ggtcttccca 1740

gggaagtacg ccatggagat gtcctccgtg gtgtacaaag gatggaaact cacggaccaa 1800

gcccttccgg tggacctcct caacaggggt gtggctgaaa aagatccatc ctcgggagag 1860

cttcggcttt tgatcgagga ttatccgtac gcggtcgacg ggctagacgt ctggaatgcc 1920

attgaggaat gggtcaagga gtactgcgca atctactacc cctcggacaa gaccctgcag 1980

gatgacaccg aggtccaagc ctggtggaag gaggtgcgtg aggtgggaca cggcgacaag 2040

aaggatgaaa catggtggcc ggccatggag accgtggctg agctgaccca gacttgctcc 2100

acgatcatct gggtggcctc cgccctccac gccgccgtca actttgcgca gtacccatac 2160

gctggttatc tgcccaaccg tccgactatt agtcgacggt tcatgcccga accgggcacg 2220

ccggagtacg aggagctaga aaccgacccc gaccgggcat tcctcaaaac gattaccagt 2280

cagctgcaga cccttatcgg tgtttctctg atcgagatcc tctccagaca ctcgtctgat 2340

gaaatctatc tagggcagag agttagcgcc gagtggactt cggacgccaa ggcgctggct 2400

gccttcaagg ctttcggtga aaagctgatc accatcgaaa acaagatcat cgagatgaac 2460

gtggactcca gcctgaagaa tcgcaacggc cccgtcaagg tcccgtacac ttttctctac 2520

cccaacacca ccgattatac ccgtgtgggt gggctgaccg gtcgtgggat ccccaacagc 2580

atctccattt ga 2592

<210> 2
<211> 1443
<212> DNA
<213> Lemna paucicostata

<400> 2
atgtctgtct cgcaatcaga tgacaaggcc gccctggtgc cgcagaagac catcccaggg 60

agctatggca tacctttcat cactcccctc aaggatcggc tcgacttctt cagcaacgaa 120

taccaattct tccagtctcg cgtagaaagc tatggctcca ccatcgtccg cctgaacgct 180

ccgccaggac ccttcatggc gaaaaaccct caagtcatcg ccattctcga cggcaagagc 240

ttccccgtcc tcttcgacac ctccaaggtc gagaaaaaga acatcttcac cggcacttac 300

atgccgtcta ctgcactcac cggcggctac cgagtttgcg cttatctcga tccgtcggag 360

cccaaccaca caaaaatcaa gcaacttctc ctgaacatcc tcttcaacag aaaagaccat 420

gtaatcccgg agttccaccg cgcgtacgaa aagctcttcg acgatatgga cgcggaaatc 480

gccaaatctg gaaaatttgt ctttaatgac cacaacgacg gtgcagcgtt cgagtttctg 540

ggcagactct tctttggagt gagcccttcg gagacggagc ttggagccgg aggagttaag 600

gacgccaatt tatggctatt ttcgcagctc tgccccctca tgaccctcgg ctttttgcct 660

aagttattgg aagatctcct cctgcacact tttcccttgc cacccttcct tttcaagggt 720

aagtaccagg cgatttacaa gtatatcagc tccgtcgcga cggacgccct aactatggcc 780

gagaatctcg ggctctcccg ggaagaagct gcccataacc tcctcttcgc cgtgtgcttt 840

aactcgctgg gcggcgtgaa agtcctcttt cccggaattc tcagatatat agcgcaagcc 900

gggaaaaatc tccaagccag ccttgtaagc gaggttcgtt ccgccgtgag ctcaactggc 960

ggggagttga cgattgaagc cctggagaag atgccgctga ccaagtcagt ggtctacgag 1020

tccctccgcc tcgatcctcc cgtcaagtac cagtacggcg tggtgaagaa ggacatggtc 1080

atcgagagcc atgacaggag ctacgaggta aaggccgggg aaacgttatt cggctaccag 1140

cctttcgcca cgagggacaa gaagattttt gggccggacg cggacaactt cgtggcggag 1200

aggttcatcg gagaagaagg cgctaaagtg ttgcggttcg ttgtgtggtc caatgggccg 1260

gagacaacag accctacgcc ggtcgataag caatgccccg ggaagaacct ggtcgtgctg 1320

atttctcggc tgctggtggc ggagtttttc ctgcgctacg acttcttcga cgcggagata 1380

ggagccgtgc cgcttgccgt caaaaccacc atcacctcct tgacgaaggc gacgacccga 1440

taa 1443

<210> 3
<211> 863
<212> PRT
<213> Lemna paucicostata

<400> 3

Met Ala Gly Phe Leu Gln Lys Val Thr Asp Asn Leu Leu His Lys Ala
1               5                   10                  15

Ala Lys Ile Ser Gly Thr Val Val Leu Val Lys Ser Asn Val Val Gly
            20                  25                  30

Phe Asn Asp Phe Ala Asp Ser Phe Leu Asp Gly Leu His Glu Leu Leu
        35                  40                  45

Gly Ser Gly Ile Thr Phe Gln Leu Val Ser Ala Thr Val Gly Asp Pro
    50                  55                  60

Lys Asn Gly Asn Lys Gly Lys Leu Gly Lys Pro Ala Leu Leu Glu Lys
65                  70                  75                  80

Trp Ile Thr Thr Gly Thr Phe Leu Ala Ala Gly Asp Ser Gly Phe Lys
                85                  90                  95

Val Asn Phe Glu Trp Asp Glu Gln Leu Gly Val Pro Gly Ala Val Ile
            100                 105                 110

Val Lys Asn Asn His His Gly Glu Phe Phe Leu Lys Ser Leu Thr Leu
        115                 120                 125

Asp Gly Ala Pro Gly Pro Arg Ser Arg Ile His Phe Asp Cys Asn Ser
    130                 135                 140

Trp Val Tyr Pro Tyr Ser Thr Tyr Asn Tyr Asp Arg Val Phe Phe Ala
145                 150                 155                 160

Asn Asp Thr Tyr Leu Pro Gly Glu Met Pro Glu Pro Leu Lys Ala Tyr
                165                 170                 175

Arg Ala Ala Glu Leu Val Asn Leu Arg Gly Asp Gly Val Thr Arg Glu
            180                 185                 190

Leu Lys Glu Gly Asp Arg Ile Tyr Ala Tyr Asp Leu Tyr Asn Asp Leu
        195                 200                 205

Gly Asp Pro Asp Ser Gly Lys Glu Leu Ala Arg Pro Ile Leu Gly Gly
    210                 215                 220

Ser Glu Glu Tyr Pro Tyr Pro Arg Arg Ala Arg Thr Gly Arg Lys Leu
225                 230                 235                 240

Thr Lys Thr Asp Pro Lys Ser Glu Gln Arg Leu Pro Leu Val Phe Ser
                245                 250                 255

Leu Asn Val Tyr Val Pro Arg Asp Glu Arg Phe Gly His Leu Lys Met
            260                 265                 270

Ser Asp Phe Leu Ala Tyr Ser Leu Lys Ala Leu Ala Gln Gly Leu Val
        275                 280                 285

Pro Ala Leu Asp Ala Ala Thr Asp Ile Thr Pro Phe Glu Phe Asp Thr
    290                 295                 300

Phe Gln Asp Val Leu Asn Leu Tyr Glu Gly Gly Ile Asn Leu Pro Thr
305                 310                 315                 320

Thr Pro Ala Leu Glu Asn Phe Lys Lys Gln Ile Pro Phe Pro Leu Val
                325                 330                 335

Lys Glu Leu Phe Arg Ser Asp Gly Glu Asn Leu Phe Arg Leu Pro Thr

340                 345                 350

Pro Ser Val Ile Lys Ala Asp Lys Phe Ala Trp Arg Thr Asp Glu Glu
        355                 360                 365

Phe Gly Arg Glu Met Leu Ala Gly Val Asn Pro Val Cys Ile Arg Leu
    370                 375                 380

Leu Lys Lys Phe Pro Pro Val Ser Ser Leu Asp Pro Ser Ile Tyr Arg
385                 390                 395                 400

Asn Gln Asn Ser Thr Met Ile Ala Asp Gln Leu Glu Lys Asn Met Asn
                405                 410                 415

Gly Leu Ser Val Glu Glu Ala Leu Lys Gln Lys Lys Leu Phe Ile Leu
            420                 425                 430

Asp His His Asp Ser Leu Met Pro Tyr Leu Glu Arg Ile Asn Thr Phe
        435                 440                 445

Asn Lys Ile Tyr Ala Ser Arg Thr Val Leu Leu Leu Gln Asp Asp Gly
    450                 455                 460

Thr Leu Lys Pro Leu Ala Ile Glu Leu Ser Leu Pro Val Lys Gly Glu
465                 470                 475                 480

Lys Gly Ala Val Ser Lys Val Tyr Thr Pro Ala Glu His Gly Val Glu
                485                 490                 495

Gly Ala Val Trp Gln Leu Ala Lys Ala Tyr Val Ala Val Asn Asp Ser
            500                 505                 510

Gly Val His Gln Leu Ile Ser His Trp Leu Asn Thr His Ala Thr Ile
        515                 520                 525

Glu Pro Phe Ser Ile Ala Leu Asn Arg Gln Leu Ser Val Val His Pro
    530                 535                 540

Ile Tyr Lys Leu Leu His Pro His Phe Arg Asp Thr Met Asn Ile Asn
545                 550                 555                 560

Ala Phe Ala Arg Gln Ile Leu Ile Asn Ala Gly Gly Ile Leu Glu Met
                565                 570                 575

Thr Val Phe Pro Gly Lys Tyr Ala Met Glu Met Ser Ser Val Val Tyr
            580                 585                 590

Lys Gly Trp Lys Leu Thr Asp Gln Ala Leu Pro Val Asp Leu Leu Asn
        595                 600                 605

Arg Gly Val Ala Glu Lys Asp Pro Ser Ser Gly Glu Leu Arg Leu Leu
    610                 615                 620

Ile Glu Asp Tyr Pro Tyr Ala Val Asp Gly Leu Asp Val Trp Asn Ala
625                 630                 635                 640

Ile Glu Glu Trp Val Lys Glu Tyr Cys Ala Ile Tyr Tyr Pro Ser Asp
                645                 650                 655

Lys Thr Leu Gln Asp Asp Thr Glu Val Gln Ala Trp Trp Lys Glu Val
            660                 665                 670

Arg Glu Val Gly His Gly Asp Lys Lys Asp Glu Thr Trp Trp Pro Ala
        675                 680                 685

Met Glu Thr Val Ala Glu Leu Thr Gln Thr Cys Ser Thr Ile Ile Trp
    690                 695                 700

Val Ala Ser Ala Leu His Ala Ala Val Asn Phe Ala Gln Tyr Pro Tyr
705                 710                 715                 720

Ala Gly Tyr Leu Pro Asn Arg Pro Thr Ile Ser Arg Arg Phe Met Pro
                725                 730                 735

Glu Pro Gly Thr Pro Glu Tyr Glu Glu Leu Glu Thr Asp Pro Asp Arg
            740                 745                 750

Ala Phe Leu Lys Thr Ile Thr Ser Gln Leu Gln Thr Leu Ile Gly Val
        755                 760                 765

Ser Leu Ile Glu Ile Leu Ser Arg His Ser Ser Asp Glu Ile Tyr Leu
    770                 775                 780

Gly Gln Arg Val Ser Ala Glu Trp Thr Ser Asp Ala Lys Ala Leu Ala
785                 790                 795                 800

Ala Phe Lys Ala Phe Gly Glu Lys Leu Ile Thr Ile Glu Asn Lys Ile
                805                 810                 815

Ile Glu Met Asn Val Asp Ser Ser Leu Lys Asn Arg Asn Gly Pro Val
            820                 825                 830

Lys Val Pro Tyr Thr Phe Leu Tyr Pro Asn Thr Thr Asp Tyr Thr Arg
        835                 840                 845

Val Gly Gly Leu Thr Gly Arg Gly Ile Pro Asn Ser Ile Ser Ile
    850                 855                 860

<210>  4
<211>  480
<212>  PRT
<213>  Lemna paucicostata

<400>  4

Met Ser Val Ser Gln Ser Asp Asp Lys Ala Ala Leu Val Pro Gln Lys
1               5                   10                  15

Thr Ile Pro Gly Ser Tyr Gly Ile Pro Phe Ile Thr Pro Leu Lys Asp
            20                  25                  30

Arg Leu Asp Phe Phe Ser Asn Glu Tyr Gln Phe Phe Gln Ser Arg Val
        35                  40                  45

Glu Ser Tyr Gly Ser Thr Ile Val Arg Leu Asn Ala Pro Pro Gly Pro
    50                  55                  60

Phe Met Ala Lys Asn Pro Gln Val Ile Ala Ile Leu Asp Gly Lys Ser
65                  70                  75                  80

Phe Pro Val Leu Phe Asp Thr Ser Lys Val Glu Lys Lys Asn Ile Phe
                85                  90                  95

Thr Gly Thr Tyr Met Pro Ser Thr Ala Leu Thr Gly Gly Tyr Arg Val
            100                 105                 110

Cys Ala Tyr Leu Asp Pro Ser Glu Pro Asn His Thr Lys Ile Lys Gln
        115                 120                 125

Leu Leu Leu Asn Ile Leu Phe Asn Arg Lys Asp His Val Ile Pro Glu
    130                 135                 140

Phe His Arg Ala Tyr Glu Lys Leu Phe Asp Asp Met Asp Ala Glu Ile
145                 150                 155                 160

Ala Lys Ser Gly Lys Phe Val Phe Asn Asp His Asn Asp Gly Ala Ala
                165                 170                 175

Phe Glu Phe Leu Gly Arg Leu Phe Phe Gly Val Ser Pro Ser Glu Thr
            180                 185                 190

Glu Leu Gly Ala Gly Gly Val Lys Asp Ala Asn Leu Trp Leu Phe Ser
        195                 200                 205

Gln Leu Cys Pro Leu Met Thr Leu Gly Phe Leu Pro Lys Leu Leu Glu
    210                 215                 220

Asp Leu Leu Leu His Thr Phe Pro Leu Pro Pro Phe Leu Phe Lys Gly

225                 230                 235                 240

Lys Tyr Gln Ala Ile Tyr Lys Tyr Ile Ser Ser Val Ala Thr Asp Ala
                245                 250                 255

Leu Thr Met Ala Glu Asn Leu Gly Leu Ser Arg Glu Glu Ala Ala His
            260                 265                 270

Asn Leu Leu Phe Ala Val Cys Phe Asn Ser Leu Gly Gly Val Lys Val
        275                 280                 285

Leu Phe Pro Gly Ile Leu Arg Tyr Ile Ala Gln Ala Gly Lys Asn Leu
    290                 295                 300

Gln Ala Ser Leu Val Ser Glu Val Arg Ser Ala Val Ser Ser Thr Gly
305                 310                 315                 320

Gly Glu Leu Thr Ile Glu Ala Leu Glu Lys Met Pro Leu Thr Lys Ser
                325                 330                 335

Val Val Tyr Glu Ser Leu Arg Leu Asp Pro Pro Val Lys Tyr Gln Tyr
            340                 345                 350

Gly Val Val Lys Lys Asp Met Val Ile Glu Ser His Asp Arg Ser Tyr
        355                 360                 365

Glu Val Lys Ala Gly Glu Thr Leu Phe Gly Tyr Gln Pro Phe Ala Thr
    370                 375                 380

Arg Asp Lys Lys Ile Phe Gly Pro Asp Ala Asp Asn Phe Val Ala Glu
385                 390                 395                 400

Arg Phe Ile Gly Glu Glu Gly Ala Lys Val Leu Arg Phe Val Val Trp
                405                 410                 415

Ser Asn Gly Pro Glu Thr Thr Asp Pro Thr Pro Val Asp Lys Gln Cys
            420                 425                 430

Pro Gly Lys Asn Leu Val Val Leu Ile Ser Arg Leu Leu Val Ala Glu
        435                 440                 445

Phe Phe Leu Arg Tyr Asp Phe Phe Asp Ala Glu Ile Gly Ala Val Pro
    450                 455                 460

Leu Ala Val Lys Thr Thr Ile Thr Ser Leu Thr Lys Ala Thr Thr Arg
465                 470                 475                 480

<210>  5
<211>  24

<212>  DNA
<213>  Artifical Sequence

<220>
<221>  misc_feature
<222>  (3)..(12)
<223>  n is inosine


<400>  5
gcntggmgna cngaygarga rtty                                              24


<210>  6
<211>  23
<212>  DNA
<213>  Artifical Sequence

<220>
<221>  misc_feature
<222>  (5)..(15)
<223>  n is inosine


<400>  6
gcrtanggrt aytgnccraa rtt                                               23


<210>  7
<211>  18
<212>  DNA
<213>  Artifical Sequence

<400>  7
agctcttcat cttggacc                                                     18


<210>  8
<211>  18
<212>  DNA
<213>  Artifical Sequence

<400>  8
tttcatcctt cttgtcgc                                                     18


<210>  9
<211>  21
<212>  DNA
<213>  Artifical Sequence

<400>  9
ggaactaacc ggaggctacc g                                                 21


<210>  10
<211>  25
<212>  DNA
<213>  Artifical Sequence

<400>  10
ccgtctccgg tccattcgac cacaa                                             25